# EUROPEAN PATENT APPLICATION

(11) **EP 4 768 576 A1**
(43) Date of publication of application: **01.07.2026**
(21) Application number: 23955862.0
(22) Date of filing: 31.10.2023
(51) Int. Cl.: C12N 9/10, C12N 15/54, C12P 19/38

(54) **PURINE NUCLEOSIDE PHOSPHORYLASE MUTANT AND PREPARATION METHOD FOR 2'-FLUORO NUCLEOSIDE**

(30) Priority: 17.10.2023 CN 202311341028
(71) Applicant: Jilin Asymchem Pharmaceuticals Co., Ltd., Dunhua, Yanbian, Jilin 133710 (CN); Tianjin Asymchem Biotechnology Co., Ltd., Tianjin 300457 (CN)
(72) Inventor: HONG, Hao, Morrisville, North Carolina 27560 (US); JAMES, Gage, Morrisville, North Carolina 27560 (US); ZHANG, Na, Tianjin 300457 (CN); JIAO, Xuecheng, Tianjin 300457 (CN); LI, Mingji, Tianjin 300457 (CN); LI, Rui, Tianjin 300457 (CN); DING, Shimao, Tianjin 300457 (CN); MI, Jiali, Tianjin 300457 (CN); GAO, Qiuyue, Tianjin 300457 (CN); DAI, Hongqian, Tianjin 300457 (CN); CUI, Yi, Tianjin 300457 (CN); CHENG, Yibing, Tianjin 300457 (CN); ZHANG, Haibin, Tianjin 300457 (CN)
(74) Representative: Zacco GmbH
(86) International application number: PCT/CN2023/128843
(87) International publication number: WO 2025/081544

(57) **Abstract**

Provide is a purine nucleoside phosphorylase mutant and a method for preparing a 2'-fluoronucleoside. The purine nucleoside phosphorylase mutant includes: (a) a protein with a mutation based on the purine nucleoside phosphorylase wild-type enzyme GsPNP as shown in SEQ ID NO: 3, where the mutation is selected from any one or more of the following mutations: L148, P19, Q41, etc.; or (b) a protein that has greater than 70% identity to the amino acid sequence as defined in (a) and has a purine nucleoside phosphorylase activity. The present application solves the problem of low yield in enzyme-catalyzed synthesis of 2'-fluoronucleoside in the prior art and is suitable for the field of enzyme catalysis.

## Description

This application is based upon and claims priority to Chinese Patent Application No. 202311341028.7, filed on October 17, 2023, the disclosure of which is hereby incorporated by reference in its entirety.

### Technical Field

The present application relates to the field of enzyme catalysis, and specifically to a purine nucleoside phosphorylase mutant and a method for preparing 2'-fluoronucleoside.

### Background

In the synthesis of nucleosides and their analogs via biotransformation, nucleoside phosphorylases can be used to convert low-cost nucleosides into high-value nucleosides. Compared with chemical synthesis, this method features mild reaction conditions, a single product, and environmental friendliness. Nucleoside phosphorylases include pyrimidine nucleoside phosphorylase (PyNP) and purine nucleoside phosphorylase (PNP). They are enzymes involved in the metabolic pathways of pyrimidines and purines, and can reversibly catalyze the phosphohydrolysis of nucleosides. Therefore, they can act synergistically and be applied to the biosynthesis of purine nucleosides and their analogs. For example, a one-pot method may be employed using PyNP and PNP to catalyze 2'-deoxyuridine and adenine to produce 2'-deoxyadenosine. PyNP can decompose 2'-deoxyuridine into 2'-deoxyribose-1-phosphate and uracil, while the other substrate, adenine, together with 2'-deoxyribose-1-phosphate generated by PyNP catalysis, can be catalyzed by PNP to synthesize 2'-deoxyadenosine.

However, for the biosynthesis of nucleoside analogs such as 2'-fluoro-2'-deoxyadenosine, since its substrate 2'-fluoro-2'-deoxyuridine is a non-natural substrate, the catalytic activity of wild-type PyNP and PNP is relatively low, thereby limiting the commercial production of 2'-fluorinated nucleoside monomers with biological methods. Zhou et al. utilized wild-type pyrimidine nucleoside phosphorylase derived from *Thermus thermophilus* (TtPyNP) and wild-type purine nucleoside phosphorylase derived from the moderately thermophilic bacterium *Deinococcus geothermalis* (DgPNP). After a 24-hour reaction, only less than 0.5 mM of 2'-fluoro-2'-deoxyadenosine could be obtained (Recombinant purine nucleoside phosphorylases from thermophiles: preparation, properties and activity towards purine and pyrimidine nucleosides). Patent CN111534493 discloses a purine nucleoside phosphorylase mutant derived from *Escherichia coli* (EcPNP), in which methionine at position 167 is mutated to arginine, and phenylalanine at position 168 is mutated to alanine. The activity of this mutant is increased by 47.6% compared with the wild-type enzyme, but the yield of 2'-fluoro-2'-deoxyadenosine prepared using this mutant is less than 1.5 mM, which cannot meet the requirements for large-scale production.

### Summary

A main objective of the present application is to provide a purine nucleoside phosphorylase mutant and a method for preparing 2'-fluoronucleoside, so as to solve the problem of low yield in enzyme-catalyzed synthesis of 2'-fluoronucleoside in the prior art.

To achieve the above objective, according to a first aspect of the present application, a purine nucleoside phosphorylase mutant is provided, including: (a) a protein with a mutation based on the purine nucleoside phosphorylase wild-type enzyme GsPNP as shown in SEQ ID NO: 3, where the mutation is selected from any one or more of the following mutations: L148, P19, Q41, T51, R56, T62, 169, S70, I73, T74, Q78, S79, Y80, N81, V82, Q83, A93, I94, K96, V98, K99, M107, T108, S109, S113, T119, Y125, A126, T128, N130, F131, K135, Y138, L142, K144, V150, S152, V153, F154, A156, F166, K168, A170, Y172, V174, L175, I205 or L206; or (b) a protein that has greater than 70% identity to the amino acid sequence as defined in (a) and has a purine nucleoside phosphorylase activity.

Further, in the (a), a type of amino acid substituted at each site is each independently selected from the following: L148P, P19M, P19K, Q41D, Q41G, Q41N, Q41T, T51F, T51W, T51Y, R56K, T62C, 169M, S70G, I73A, 173G, I73L, T74Q, Q78A, Q78K, S79T, Y80A, Y80P, Y80T, N81G, V82A, Q83G, A93S, 194A, 194L, 194N, K96E, K96P, K96S, V98E, V98H, V98L, V98M, V98Q, V98S, V98T, K99S, M107E, M107G, M107H, M107N, M107S, M107Y, T108I, T108L, T108R, T108V, S109N, S113N, T119C, T119K, Y125F, Y125H, Y125K, Y125M, Y125Y, A126G, A126S, T128C, N130D, F131A, F131I, F131L, F131P, F131S, F131T, F131W, K135E, Y138S, Y138L, K142R, K144A, K144L, L148A, L148G, L148Q, L148S, L148Y, V150E, V150P, S152D, S152G, S152H, S152K, S152N, S152P, V153S, F154L, F154K, A156E, F166I, F166P, F166S, F166T, F166V, K168E, A170E, A170R, Y172A, Y172C, Y172G, Y172L, Y172N, Y172Q, Y172R, V174D, V174G, V174L, V174M, V174N, V174Q, V174S, V174T, L175A, L175E, L175G, L175K, L175N, L175S, L175T, 1205A, I205V or L206I; where a letter in front of a number represents an original amino acid, and a letter behind the number represents a mutated amino acid.

Further, the above mutation includes any one of the following amino acid mutations: L148P+P19M, L148P+P19K, L148P+Q41D, L148P+Q41G, L148P+Q41N, L148P+Q41T, L148P+T51F, L148P+T51W, L148P+T51Y, L148P+R56K, L148P+T62C, L148P+I69M, L148P+S70G, L148P+173A, L148P+I73G, L148P+I73L, L148P+T74Q, L148P+Q78A, L148P+Q78K, L148P+S79T, L148P+Y80A, L148P+Y80P, L148P+Y80T, L148P+N81G, L148P+V82A, L148P+Q83G, L148P+A93S, L148P+I94A, L148P+I94L, L148P+194N, L148P+K96E, L148P+K96P, L148P+K96S, L148P+V98E, L148P+V98H, L148P+V98L, L148P+V98M, L148P+V98Q, L148P+V98S, L148P+V98T, L148P+K99S, L148P+M107E, L148P+M107G, L148P+M107H, L148P+M107N, L148P+M107S, L148P+M107Y, L148P+T108I, L148P+T108L, L148P+T108R, L148P+T108V, L148P+S109N, L148P+S113N, L148P+T119C, L148P+T119K, L148P+Y125F, L148P+Y125H, L148P+Y125K, L148P+Y125M, L148P+Y125Y, L148P+A126G, L148P+A126S, L148P+T128C, L148P+N130D, L148P+F131A, L148P+F131I, L148P+F131L, L148P+F131P, L148P+F131S, L148P+F131T, L148P+F131W, L148P+K135E, L148P+Y138S, L148P+Y138L, L148P+K142R, L148P+K144A, L148P+K144L, L148P+V150E, L148P+V150P, L148P+S152D, L148P+S152G, L148P+S152H, L148P+S152K, L148P+S152N, L148P+S152P, L148P+V153S, L148P+F154L, L148P+F154K, L148P+A156E, L148P+F166I, L148P+F166P, L148P+F166S, L148P+F166T, L148P+F166V, L148P+K168E, L148P+A170E, L148P+A170R, L148P+Y172A, L148P+Y172C, L148P+Y172G, L148P+Y172L, L148P+Y172N, L148P+Y172Q, L148P+Y172R, L148P+V174D, L148P+V174G, L148P+V174L, L148P+V174M, L148P+V174N, L148P+V174Q, L148P+V174S, L148P+V174T, L148P+L175A, L148P+L175E, L148P+L175G, L148P+L175K, L148P+L175N, L148P+L175S, L148P+L175T, L148P+I205A, L148P+I205V, L148P+L206I, L148P+P19K+M107G, L148P+P19M+F166P, L148P+Q41G+T119K, L148P+Q41D+A156E, L148P+Q41N+K96E, L148P+Q41T+T51W, L148P+Q41T+N81G, L148P+Q41T+T108R, L148P+Q41T+L175K, L148P+T51F+F166I, L148P+T51W+V174S, L148P+T51Y+F166P, L148P+R56K+A126S, L148P+T62C+M107H, L148P+I69M+K135E, L148P+S70G+Y125H, L148P+173A+S152N, L148P+I73A+M107Y, L148P+173G+V98Q, L148P+T74Q+I94N, L148P+Q78A+V174S, L148P+Q78K+L175G, L148P+S79T+A93S, L148P+Y80A+F154L, L148P+Y80P+Y172A, L148P+Y80T+L206I, L148P+Q83G+S152D, L148P+V98E+V153S, L148P+V98L+V174L, L148P+V98M+F131I, L148P+V98H+K168E, L148P+V98S+Y172Q, L148P+M107S+S152K, L148P+T108L+V174D, L148P+T108V+L175K, L148P+S113N+S152H, L148P+T119C+S152G, L148P+Y125M+Y172L, L148P+A126G+Y172G, L148P+T128C+V174N, L148P+N130D+V150E, L148P+F131L+I205V, L148P+F131P+Y172C, L148P+F131W+L175S, L148P+Y138S+V174Q, L148P+F166S+V174M, L148P+F166V+L175E, L148P+P19K+T108V+Y125Y, L148P+P19M+M107N+F166P, L148P+Q41D+M107G+S152P, L148P+Q41N+Y80P+K144A, L148P+Q41T+M107H+V174L, L148P+Q41T+L175K+I73A, L148P+T51W+Y80P+V174S, L148P+T51Y+F131T+V174G, L148P+R56K+Q78A+F166S, L148P+T62C+Y125K+V150P, L148P+I73A+T108L+F131S, L148P+I73G+K96P+S1562H, L148P+I73L+V98M+FI66P, L148P+V82A+A93S+F131L, L148P+I69M+A156E+L175E, L148P+S70G+S113N+Y172R, L148P+S79T+A126G+S152D, L148P+I94A+F131P+V174M, L148P+194L+Y138L+V174T, L148P+194N+N130D+L206I, L148P+K96E+F131W+F166I, L148P+V98H+T119K+F166T, L148P+V98Q+Y138S+A170E, L148P+V98L+K135E+V174L, L148P+M107E+S152N+L175K, L148P+T108R+A126S+K168E, L148P+S109N+F131I+F154L, L148P+T119C+Y125H+K135E, L148P+F131A+K142R+V174S, L148P+S152G+F166V+I205V, L148P+P19K+K96P+V150P+A126S, L148P+P19M+M 107N+F166P+V174T, L148P+Q41D+T108L+K144A+V153S, L118P+Q41G+T51Y+S113N+F166I, L148P+Q41N+T51F+A93S+F131A, L148P+Q41N+Y80A+S152G+L175E, L148P+Q41T+Y80A+V150E+Y172A, L148P+Q41T+173A+S113N+L175K, L148P+T51W+Y80P+Y125M+V174S, L148P+T51Y+173A+Y125K+L142R, L148P+R56K+M107H+A126G+L206I, L148P+T62C+V98Q+M107G+S152P, L148P+169M+F131W+L175K+L206I, L148P+173G+F131L+S152K+Y172C, L148P+173L+194A+T119C+S152D, L148P+T74Q+V98S+T108I+I205V, L148P+Q78A+S152P+F166V+L175G, L148P+Q78K+I94L+L175E+L206I, L148P+S79T+K96E+Y138L+V174M, L148P+Y80T+M107S+S152H+Y172R, L148P+N81G+Y125Y+F131T+A156E, L148P+Q83G+M107Y+A126S+K168E, L148P+V98L+T119K+K135E+V174L, L148P+V98T+M107E+Y138S+I205A, L148P+Y125H+F131I+S152H+L175T, L148P+P19M+T51Y+M107N+F166P+V174T, L148P+Q41T+I73A+A126S+L175K+S113N, L148P+T51W+Y80P+Y125M+S152P+V174S, L148P+I73L+Y80T+M107S+S152H+Y172R, L148P+Q78A+S152P+F166V+L175G+I205V, L148P+VI8L+T119K+K135E+F166P+V174L, L148P+P19M+T51Y+M107N+S152H+F166P+V174T, L148P+Q41D+V98L+T119K+K135E+F166P+V174L, L148P+Q41T+I73A+A126S+K144A+L175K+S113N, L148P+T51W+Y80P+M107N+S113N+F166V+l205A, L148P+T51W+Y80P+Y125M+S152P+V174S+L206I, L148P+R56K+I73L+Y80T+M107S+S152H+Y172R, L148P+Q78A+M107S+S152P+F166V+L175G+I205V, L148P+P19M+T51Y+I73A+M107N+S152H+F166P+V174T, L148P+Q41D+V98L+T119K+K135E+S152H+F166P+V174L, L148P+Q41T+173A+S113N+A126S+K144A+L175K+I205V, L148P+T51W+Y80P+M107N+S113N+F166V+L175G+I205A, L148P+T51W+Y80P+V98L+Y125M+S152P+V174S+L206I, L148P+R56K+I73L+Y80T+M107S+T119K+S152H+Y172R, L148P+Q78A+M107S+Y125H+S152P+F166V+L175G+I205V, L148P+P19M+Q41T+I73A+S113N+A126S+K144A+L175K+I205V, L148P+P19M+T51Y+173A+V98T+M107N+S152H+F166P+V174T, L148P+Q41D+V98L+T119K+K135E+S152H+F166P+V174L+I205V, L148P+T51W+Y80P+V98T+M107N+S113N+F166V+L175G+I205A, L148P+T51W+Y80P+V98L+Y125M+S152P+F166V+V174S+L206I, L148P+R56K+I73L+Y80T+M107S+T119K+S152H+Y172R+L206I, L148P+Q78A+M107S+S113N+Y125H+S152P+F166V+L175G+I205V, L148P+P19M+Q41T+I69M+I73A+S113N+A126S+K144L+L175K+I205V, L148P+P19M+T51Y+I73A+VI8T+M107N+S152H+F166P+V174T+L206I, L148P+Q41D+S70G+V98L+T119K+K135E+S152H+F166P+V174L+I205V, L148P+T51W+I69M+Y80P+V98T+M107N+S113N+F166V+L175G+I205A, L148P+T51W+Y80P+V98L+Y125M+K144L+S152P+F166V+V174S+L2061, L148P+R56K+173L+Y80T+M107S+T119K+K144A+S152H+Y172R+L2061, L148P+Q78A+V98L+M107S+S113N+Y125H+S152P+F166V+L175G+I205V, L148P+P19M+Q41T+I69M+I73A+S113N+Y125F+A126S+T128C+N130D+K144L+L175K+I205V, L148P+P19M+Q41D+T51Y+I73A+V98T+M107N+A126G+K135E+S152H+F166P+V174T+L206I, L148P+Q41D+S70G+V98L+T108L+T119K+Y125F+K135E+K144A+S152H+F166P+V174L+I205V, L148P+T51W+169M+Y80P+V98T+M107N+S113N+N130D+K142R+F154L+F166V+L175G+I205A, L148P+T51W+T74Q+Y80P+V98L+T108I+S113N+Y125M+K144L+S152P+F166V+V174S+L206I, L148P+R56K+I73L+Y80T+VI8M+M107S+T119K+Y125F+F131S+K144A+S152H+Y172R+L206I, or L148P+I69M+Q78A+V98L+M107S+S113N+Y125H+N130D+K144A+S152P+F166V+L175G+I205 V.

Further, the purine nucleoside phosphorylase mutant includes a protein that has greater than 80%, greater than 85%, more preferably greater than 95%, and further preferably greater than 99% identity to the amino acid sequence as defined in (a) and has a purine nucleoside phosphorylase activity;
Preferably, the purine nucleoside phosphorylase mutant includes a protein as shown in SEQ ID NO: 5, or a protein that has greater than 80% identity to the protein as shown in SEQ ID NO: 5 and has a purine nucleoside phosphorylase activity.

To achieve the above objective, according to a second aspect of the present application, a DNA molecule is provided, where the DNA molecule encodes the above purine nucleoside phosphorylase mutant.

To achieve the above objective, according to a third aspect of the present application, a recombinant plasmid is provided, where the above DNA molecule is inserted into the recombinant plasmid.

To achieve the above objective, according to a fourth aspect of the present application, a host cell is provided, where the host cell includes the above DNA molecule or recombinant plasmid.

Further, the host cell includes a prokaryotic cell; and preferably, the prokaryotic cell includes *Escherichia coli.*

To achieve the above objective, according to a fifth aspect of the present application, a method for preparing a 2'-fluoronucleoside is provided, where the method includes: catalyzing the binding of a phosphorylated 2'-fluoropentose to a substrate base with the above purine nucleoside phosphorylase, so as to obtain the 2'-fluoronucleoside.

Further, a method for preparing the phosphorylated 2'-fluoronucleoside includes: catalyzing the cleavage of a substrate nucleoside having a 2'-fluoronucleoside structure into a phosphorylated 2'-fluoropentose and a free base with a nucleoside phosphorylase; and preferably, the nucleoside phosphorylase includes a pyrimidine nucleoside phosphorylase, a thymidine phosphorylase, a uridine phosphorylase or a 5'-methylthioadenosine phosphorylase.

Further, the substrate nucleoside includes a 2'-fluoro-2'-deoxyuridine, a 2'-fluoro-2'-deoxycytidine or a 2'-deoxy-2'-fluorothymidine.

Further, the substrate base includes a modified or unmodified adenine, guanine, thymine, cytosine, or a modified uracil; and preferably, the substrate base includes an adenine, a guanine or a 2-aminoadenine.

Further, the 2'-fluoronucleoside includes a nucleoside containing a 2'-fluoro-2'-deoxypentose structure; and preferably, the 2'-fluoronucleoside includes a 2'-fluoro-2'-deoxyadenosine, a 2'-fluoro-2'-deoxy-2-aminoadenosine, a 2'-fluoro-2'-deoxyguanosine or a 2'-fluoro-2'-deoxycytidine; or the 2'-fluoronucleoside is selected from a 2'-fluoro-2'-deoxynucleoside with a substituent on the base structure.

By applying the technical solutions of the present application, the binding of phosphorylated 2'-fluoropentose and different substrate bases can be efficiently catalyzed with the above purine nucleoside phosphorylase mutant to prepare and obtain the 2'-fluoronucleoside. Compared with the wild-type enzyme, the above purine nucleoside phosphorylase mutant has a better enzyme activity, and a higher conversion rate and greater efficiency in preparing 2'-fluoronucleoside products. It can significantly reduce the amount of enzyme used, thereby lowering production costs, and is suitable for industrial scale-up production.

### Brief Description of the Drawings

The drawings that form part of this application are used to provide a further understanding of the present application. The schematic examples of the present application and their descriptions are used to explain the present application, and do not constitute an improper limitation of the present application. In the drawings:
Fig. 1 shows a schematic diagram of the chemical reaction in which a phosphorylated 2'-fluoropentose and a substrate base are catalyzed to combine to prepare and obtain 2'-fluoronucleoside with a purine nucleoside phosphorylase according to an example of the present application.
Fig. 2 shows a schematic diagram of a chemical reaction in which a substrate nucleoside with a 2'-fluoropentose is catalyzed to cleave into a phosphorylated 2'-fluoropentose and a free base according to an example of the present application.
Fig. 3 shows an HPLC chromatogram of preparing 2'-fluoro-2'-deoxyadenosine with 2'-fluoro-2'-deoxyuridine and adenine as substrates according to Example 4 of the present application.

### Detailed Description of the Embodiments

It should be noted that, in the absence of a conflict, embodiments of this application can be combined with features in the embodiments. The present application will be described in detail below in conjunction with the embodiments.

### Terminology explanation:

2'-fluoronucleoside: a nucleoside in which the hydroxyl group on the 2-position carbon atom of the pentose is replaced by a fluorine atom.

As mentioned in the background, the yield and output of preparing 2'-fluoronucleoside with enzyme catalysis are relatively low, which is difficult to meet the requirements of industrial production. Therefore, in this application, the inventors attempt to develop a purine nucleoside phosphorylase mutant with a higher enzyme activity. Through directed evolution, a purine nucleoside phosphorylase mutant is obtained whose catalytic activity is improved by approximately 20 times compared with the wild-type, which reduces the amount of enzyme used, thereby lowering production costs and enabling application in large-scale production of related products. This allows for the efficient preparation of the target product, 2'-fluoronucleoside, and thus a series of protection schemes are proposed in this application.

In a first typical implementation of this application, a purine nucleoside phosphorylase mutant is provided, including: (a) a protein with a mutation based on the purine nucleoside phosphorylase wild-type enzyme GsPNP as shown in SEQ ID NO: 3, where the mutation is selected from any one or more of the following mutations: L148, P19, Q41, T51, R56, T62, 169, S70, 173, T74, Q78, S79, Y80, N81, V82, Q83, A93, 194, K96, V98, K99, M107, T108, S109, S113, T119, Y125, A126, T128, N130, F131, K135, Y138, L142, K144, V150, S152, V153, F154, A156, F166, K168, A170, Y172, V174, L175, I205 or L206; (b) a protein that has greater than 70% identity to the amino acid sequence as defined in (a) and has a purine nucleoside phosphorylase activity.

In a preferred embodiment, in the (a), a type of amino acid substituted at each site is each independently selected from the following:
L148P, P19M, P19K, Q41D, Q41G, Q41N, Q41T, T51F, T51W, T51Y, R56K, T62C, 169M, S70G, 173A, 173G, I73L, T74Q, Q78A, Q78K, S79T, Y80A, Y80P, Y80T, N81G, V82A, Q83G, A93S, I94A, 194L, 194N, K96E, K96P, K96S, V98E, V98H, V98L, V98M, V98Q, V98S, V98T, K99S, M107E, M107G, M107H, M107N, M107S, M107Y, T108I, T108L, T108R, T108V, S109N, S113N, T119C, T119K, Y125F, Y125H, Y125K, Y125M, Y125Y, A126G, A126S, T128C, N130D, F131A, F131I, F131L, F131P, F131S, F131T, F131W, K135E, Y138S, Y138L, K142R, K144A, K144L, L148A, L148G, L148Q, L148S, L148Y, V150E, V150P, S152D, S152G, S152H, S152K, S152N, S152P, V153S, F154L, F154K, A156E, F166I, F166P, F166S, F166T, F166V, K168E, A170E, A170R, Y172A, Y172C, Y172G, Y172L, Y172N, Y172Q, Y172R, V174D, V174G, V174L, V174M, V174N, V174Q, V174S, V174T, L175A, L175E, L175G, L175K, L175N, L175S, L175T, 1205A, I205V or L206I; where a letter in front of a number represents an original amino acid, and a letter behind the number represents a mutated amino acid.

In a preferred embodiment, the above mutation includes any one of the following amino acid mutations: L148P+P19M, L148P+P19K, L148P+Q41D, L148P+Q41G, L148P+Q41N, L148P+Q41T, L148P+T51F, L148P+T51W, L148P+T51Y, L148P+R56K, L148P+T62C, L148P+I69M, L148P+S70G, L148P+173A, L148P+I73G, L148P+I73L, L148P+T74Q, L148P+Q78A, L148P+Q78K, L148P+S79T, L148P+Y80A, L148P+Y80P, L148P+Y80T, L148P+N81G, L148P+V82A, L148P+Q83G, L148P+A93S, L148P+I94A, L148P+I94L, L148P+194N, L148P+K96E, L148P+K96P, L148P+K96S, L148P+V98E, L148P+V98H, L148P+V98L, L148P+V98M, L148P+V98Q, L148P+V98S, L148P+V98T, L148P+K99S, L148P+M107E, L148P+M107G, L148P+M107H, L148P+M107N, L148P+M107S, L148P+M107Y, L148P+T108I, L148P+T108L, L148P+T108R, L148P+T108V, L148P+S109N, L148P+S113N, L148P+T119C, L148P+T119K, L148P+Y125F, L148P+Y125H, L148P+Y125K, L148P+Y125M, L148P+Y125Y, L148P+A126G, L148P+A126S, L148P+T128C, L148P+N130D, L148P+F131A, L148P+F131I, L148P+F131L, L148P+F131P, L148P+F131S, L148P+F131T, L148P+F131W, L148P+K135E, L148P+Y138S, L148P+Y138L, L148P+K142R, L148P+K144A, L148P+K144L, L148P+V150E, L148P+V150P, L148P+S152D, L148P+S152G, L148P+S152H, L148P+S152K, L148P+S152N, L148P+S152P, L148P+V153S, L148P+F154L, L148P+F154K, L148P+A156E, L148P+F166I, L148P+F166P, L148P+F166S, L148P+F166T, L148P+F166V, L148P+K168E, L148P+A170E, L148P+A170R, L148P+Y172A, L148P+Y172C, L148P+Y172G, L148P+Y172L, L148P+Y172N, L148P+Y172Q, L148P+Y172R, L148P+V174D, L148P+V174G, L148P+V174L, L148P+V174M, L148P+V174N, L148P+V174Q, L148P+V174S, L148P+V174T, L148P+L175A, L148P+L175E, L148P+L175G, L148P+L175K, L148P+L175N, L148P+L175S, L148P+L175T, L148P+I205A, L148P+I205V, L148P+L206I, L148P+P19K+M107G, L148P+P19M+F166P, L148P+Q41G+T119K, L148P+Q41D+A156E, L148P+Q41N+K96E, L148P+Q41T+T51W, L148P+Q41T+N81G, L148P+Q41T+T108R, L148P+Q41T+L175K, L148P+T51F+F166I, L148P+T51W+V174S, L148P+T51Y+F166P, L148P+R56K+A126S, L148P+T62C+M107H, L148P+I69M+K135E, L148P+S70G+Y125H, L148P+173A+S152N, L148P+I73A+M107Y, L148P+173G+V98Q, L148P+T74Q+I94N, L148P+Q78A+V174S, L148P+Q78K+L175G, L148P+S79T+A93S, L148P+Y80A+F154L, L148P+Y80P+Y172A, L148P+Y80T+L206I, L148P+Q83G+S152D, L148P+V98E+V153S, L148P+V98L+V174L, L148P+V98M+F131I, L148P+V98H+K168E, L148P+V98S+Y172Q, L148P+M107S+S152K, L148P+T108L+V174D, L148P+T108V+L175K, L148P+S113N+S152H, L148P+T119C+S152G, L148P+Y125M+Y172L, L148P+A126G+Y172G, L148P+T128C+V174N, L148P+N130D+V150E, L148P+F131L+I205V, L148P+F131P+Y172C, L148P+F131W+L175S, L148P+Y138S+V174Q, L148P+F166S+V174M, L148P+F166V+L175E, L148P+P19K+T108V+Y125Y, L148P+P19M+M107N+F166P, L148P+Q41D+M107G+S152P, L148P+Q41N+Y80P+K144A, L148P+Q41T+M107H+V174L, L148P+Q41T+L175K+I73A, L148P+T51W+Y80P+V174S, L148P+T51Y+F131T+V174G, L148P+R56K+Q78A+F166S, L148P+T62C+Y125K+V150P, L148P+I73A+T108L+F131S, L148P+I73G+K96P+S1562H, L148P+I73L+V98M+F166P, L148P+V82A+A93S+F131L, L148P+I69M+A156E+L175E, L148P+S70G+S113N+Y172R, L148P+S79T+A126G+S152D, L148P+I94A+F131P+V174M, L148P+I94L+Y138L+V174T, L148P+I94N+N130D+L206I, L148P+K96E+F131W+F166I, L148P+V98H+T119K+F166T, L148P+V98Q+Y138S+A170E, L148P+V98L+K135E+V174L, L148P+M107E+S152N+L175K, L148P+T108R+A126S+K168E, L148P+S109N+F131I+F154L, L148P+T119C+Y125H+K135E, L148P+F131A+K142R+V174S, L148P+S152G+F166V+I205V, L148P+P19K+K96P+V150P+A126S, L148P+P19M+M107N+F166P+V174T, L148P+Q41D+T108L+K144A+V153S, L148P+Q41G+T51Y+S113N+F166I, L148P+Q41N+T51F+A93S+F131A, L148P+Q41N+Y80A+S152G+L175E, L148P+Q41T+Y80A+V150E+Y172A, L148P+Q41T+I73A+S113N+L175K, L148P+T51W+Y80P+Y125M+V174S, L148P+T51Y+173A+Y125K+L142R, L148P+R56K+M107H+A126G+L206I, L148P+T62C+V98Q+M107G+S152P, L148P+169M+F131W+L175K+L206I, L148P+173G+F131L+S152K+Y172C, L14SP+I73L+I94A+T119C+S152D, L148P+T74Q+V98S+T108I+I205V, L148P+Q78A+S152P+F166V+L175G, L148P+Q78K+l94L+L175E+L206I, L148P+S79T+K96E+Y138L+V174M, L148P+Y80T+M107S+S152H+Y172R, L148P+N81G+Y125Y+F131T+A156E, L148P+Q83G+M107Y+A126S+K168E, L148P+V98L+T119K+K135E+V174L, L148P+V98T+M107E+Y138S+I205A, L148P+Y125H+F131I+S152H+L175T, L148P+P19M+T51Y+M107N+F166P+V174T, L148P+Q41T+I73A+A126S+L175K+S113N, L148P+T51W+Y80P+Y125M+S152P+V174S, L148P+I73L+Y80T+M107S+S152H+Y172R, L148P+Q78A+S152P+F166V+L175G+I205V, L148P+V98L+T119K+K135E+F166P+V174L, L148P+P19M+T51Y+M107N+S152H+F166P+V174T, L148P+Q41D+V9SL+T119K+K135E+F166P+V174L, L148P+Q41T+I73A+A126S+K144A+L175K+S113N, L148P+T51W+Y80P+M107N+S113N+F166V+I205A, L148P+T51W+Y80P+Y125M+S152P+V174S+L206I, L148P+R56K+I73L+Y80T+M107S+S152H+Y172R, L148P+Q78A+M107S+S152P+F166V+L175G+I205V, L148P+P19M+T51Y+I73A+M107N+S152H+F166P+V174T, L148P+Q41D+V98L+T119K+K135E+S152H+F166P+V174L, L148P+Q41T+173A+S113N+A126S+K144A+L175K+I205V, L148P+T51W+Y80P+M107N+S113N+F166V+L175G+I205A, L148P+T51W+Y80P+V98L+Y125M+S152P+V174S+L206I, L148P+R56K+I73L+Y80T+M107S+T119K+S152H+Y172R, L148P+Q78A+M107S+Y125H+S152P+F166V+L175G+I205V, L148P+P19M+Q41T+I73A+S113N+A126S+K144A+L175K+I205V, L148P+P19M+T51Y+I73A+V98T+M107N+S152H+F166P+V174T, L148P+Q41D+V98L+T119K+K135E+S152H+F166P+V174L+I205V, L148P+T51W+Y80P+V98T+M107N+S113N+F166V+L175G+I205A, L148P+T51W+Y80P+V98L+Y125M+S152P+F166V+V174S+L206I, L148P+R56K+I73L+Y80T+M107S+T119K+S152H+Y172R+L206l, L148P+Q78A+M107S+S113N+Y125H+S152P+F166V+L175G+I205V, L148P+P19M+Q41T+I69M+I73A+S113N+A126S+K144L+L175K+I205V, L148P+P19M+T51Y+I73A+V98T+M107N+S152H+F166P+V174T+L206I, L148P+Q41D+S70G+V98L+T119K+K135E+S152H+F166P+V174L+I205V, L148P+T51W+I69M+Y80P+V98T+M107N+S113N+F166V+L175G+I205A, L148P+T51W+Y80P+V98L+Y125M+K144L+S152P+F166V+V174S+L206I, L148P+R56K+173L+Y80T+M107S+T119K+K144A+S152H+Y172R+L206I, L148P+Q78A+V98L+M107S+S113N+Y125H+S152P+F166V+L175G+I205V, L148P+P19M+Q41T+I69M+I73A+S113N+Y125F+A126S+T128C+N130D+K144L+L175K+I205V, L148P+P19M+Q41D+T51Y+I73A+VI8T+M107N+A126G+K135E+S1562H+F166P+V174T+L206I, L148P+Q41D+S70G+V98L+T108L+T119K+Y125F+K135E+K144A+S152H+F166P+V174L+I205V, L148P+T51W+169M+Y80P+V98T+M107N+S113N+N130D+K142R+F154L+F166V+L175G+I205A, L148P+T51W+T74Q+Y80P+V98L+T108I+S113N+Y125M+K144L+S152P+F166V+V174S+L206I, L148P+R56K+I73L+Y80T+VI8M+M107S+T119K+Y125F+F131S+K144A+S152H+Y172R+L206I, or L148P+I69M+Q78A+V98L+M107S+S113N+Y125H+N130D+K144A+S152P+F166V+L175G+I205 V.

SEQ ID NO: 3:

MSVHIGAKEHEIADKILLPGDPLRAKYIAETFLEGATCYNQVRGMLGFTGTYKGHRISVQGTG
MGVPSISIYITELMQSYNVQTLIRVGTCGAIQKDVKVRDVILAMTSSTDSQMNRMTFGGIDYAPTAN
FDLLKTAYEIGKEKGLQLKVGSVFTADMFYNENAQFEKLARYGVLAVEMETTALYTLAAKFGRKALS
VLTVSDHILTGEETTAEERQTTFNEMIEVALETAIRQ.

In a preferred embodiment, the purine nucleoside phosphorylase mutant includes a protein that has greater than 80%, greater than 85%, more preferably greater than 95%, and further preferably greater than 99% identity to the amino acid sequence as defined in (a), and has a purine nucleoside phosphorylase activity; and preferably, the purine nucleoside phosphorylase mutant includes the protein as shown in SEQ ID NO: 5, or a protein that has greater than 70%, 75%, 80%, 85%, 90%, 91%, 92%, 93%, 94%, 95%, 96%, 97%, 98%, 99%, 99.5%, 99.7%, 99.8% or 99.9% identity to the protein as shown in SEQ ID NO: 5 and has a purine nucleoside phosphorylase activity.

SEQ ID NO: 5:

MSVHIGAKEHEIADKILLMGDPLRAKYIAETFLEGATCYNTVRGMLGFTGTYKGHRISVQGTG
MGVPSMSIYATELMQSYNVQTLIRVGTCGAIQKDVKVRDVILAMTSSTDNQMNRMTFGGIDFSPCA
DFDLLKTAYEIGKELGLQPKVGSVFTADMFYNENAQFEKLARYGVKAVEMETTALYTLAAKFGRKA
LSVLTVSDHVLTGEETTAEERQTTFNEMIEVALETAIRQ.

All the above amino acid mutations have been experimentally investigated in the examples of this application. Compared with the parent having the amino acid sequence as shown in SEQ ID NO: 3, all of them have the activity of catalyzing the binding of a phosphorylated 2'-fluoropentose and a substrate base to prepare and obtain a 2'-fluoronucleoside. All the above mutation sites are mutations around the amino acid active site. Such mutations can improve the binding ability and/or catalytic ability of the mutant with the substrate. For mutations far from the active site, they have little impact on the catalytic ability of the enzyme. Therefore, a protein that has 80% or more identity to the above amino acid sequence and the same catalytic activity can be obtained.

"Identity" in this specification refers to the "identity" between amino acid sequences, i.e., the total ratio of amino acid residues of the same type in the amino acid sequences. The identity of amino acid sequences can be determined using alignment programs such as BLAST (Basic Local Alignment Search Tool), FASTA, and the like.

A protein that has greater than 70%, 75%, 80%, 85%, 90%, 95%, 99% (such as greater than 85%, 86%, 87%, 88%, 89%, 90%, 91%, 92%, 93%, 94%, 95%, 96%, 97%, 98%, 98.5%, 99%, 99.5%, 99.6%, 99.7%, 99.8%, and even greater than 99.9%) identity and the same function is highly likely to have the same active site, active pocket, active mechanism, protein structure, etc. as the protein provided by the sequence in (a), and is a homologous protein obtained through amino acid mutation.

As used herein, the abbreviations of amino acid residues are as follows: alanine (Ala; A), asparagine (Asn; N), aspartic acid (Asp; D), arginine (Arg; R), cysteine (Cys; C), glutamic acid (Glu; E), glutamine (Gln; Q), glycine (Gly; G), histidine (His; H), isoleucine (Ile; I), leucine (Leu; L), lysine (Lys; K), methionine (Met; M), phenylalanine (Phe; F), proline (Pro; P), serine (Ser; S), threonine (Thr; T), tryptophan (Trp; W), tyrosine (Tyr; Y), and valine (Val; V).

According to the rules of substitution and replacement, in general, the effect of mutual replacement between amino acids with similar properties is also similar. For example, in the above homologous proteins, conservative amino acid replacements may occur. "Conservative amino acid replacements" include but are not limited to:
Hydrophobic amino acids (Ala, Cys, Gly, Pro, Met, Val, Ile, Leu) substituted by other hydrophobic amino acids;
Hydrophobic amino acids with bulky side chains (Phe, Tyr, Trp) substituted by other hydrophobic amino acids with bulky side chains;
Amino acids with positively charged side chains (Arg, His, Lys) substituted by other amino acids with positively charged side chains; and
Amino acids with polar but uncharged side chains (Ser, Thr, Asn, Gln) substituted by other amino acids with polar but uncharged side chains.

A person skilled in the art can also perform conservative replacements of amino acids according to amino acid replacement rules well-known in the art, such as the "blosum62 scoring matrix" in the prior art.

"AlphaFold2-Multimer" used in this application is a publicly available artificial intelligence model capable of predicting the conformation of protein complexes. Its prediction of protein three-dimensional structure can be very close to the level observed with equipment such as cryo-electron microscopy in real experiments. It can obtain a relatively realistic protein structure, thereby guiding the exploration of protein structure and protein activity.

In a second typical implementation of this application, a DNA molecule is provided, where the DNA molecule encodes the above purine nucleoside phosphorylase mutant.

Preferably, the DNA molecule includes the nucleotide sequence as shown in SEQ ID NO: 6.

SEQ ID NO: 6:

ATGAGCGTGCATATTGGCGCGAAAGAACATGAAATTGCGGATAAAATTCTGCTGATGGGCG
ATCCGTTACGCGCGAAATATATTGCGGAAACCTTTCTGGAAGGCGCGACCTGCTATAACACCGT
GCGCGGCATGCTGGGCTTTACCGGCACCTATAAAGGCCATCGCATTAGCGTGCAGGGCACCG
GCATGGGCGTTCCTAGCATGTCAATTTATGCTACCGAACTGATGCAGAGCTATAACGTGCAGAC
CCTGATTCGCGTGGGCACCTGCGGTGCGATTCAAAAAGATGTGAAAGTGCGCGATGTGATTCT
GGCGATGACCAGCAGCACCGATAACCAGATGAACCGCATGACCTTTGGCGGCATTGATTTTAG
TCCGTGCGCGGACTTTGATCTGCTGAAAACCGCGTATGAAATTGGCAAAGAACTGGGCCTGCA
GCCGAAAGTGGGCAGCGTGTTTACCGCGGATATGTTTTATAACGAAAACGCGCAGTTTGAGAA
ACTGGCCCGCTATGGCGTGAAGGCGGTTGAAATGGAAACCACCGCGCTGTATACCCTGGCGG
CGAAATTTGGCCGCAAAGCGCTGAGCGTGCTGACCGTGAGCGATCATGTTCTGACCGGCGAA
GAAACCACCGCGGAAGAACGCCAGACCACCTTTAACGAAATGATTGAAGTGGCGCTGGAAAC
CGCGATTCGCCAGTAA.

In a third typical implementation of this application, a recombinant plasmid is provided, where the above DNA molecule is comprised in the recombinant plasmid.

The above DNA can encode the above purine nucleoside phosphorylase mutant, and can be linked to the recombinant plasmid to form circular DNA. Both the above DNA and recombinant plasmid can undergo transcription and translation under the action of RNA polymerase, ribosomes, tRNA, etc., to obtain the above purine nucleoside phosphorylase mutant.

In a fourth typical implementation of this application, a host cell is provided, where the host cell includes the above DNA molecule or the above recombinant plasmid.

With the above host cell, the replication of the recombinant plasmid can be carried out in the host cell, and the DNA molecule carried on the recombinant plasmid can also be transcribed and translated to obtain a large amount of purine nucleoside phosphorylase mutants. With the prior art, such as cell disruption for protein purification of host cells, crude enzyme catalysis after disruption, or other methods, the purine nucleoside phosphorylase mutant can be obtained for subsequent catalysis of the substrate nucleoside. The host cell is a non-plant-derived host cell.

In a preferred embodiment, the host cell includes a prokaryotic cell; and preferably, the prokaryotic cell includes *Escherichia coli.*

In a fifth typical implementation of this application, a method for preparing a 2'-fluoronucleoside is provided, where the method includes: catalyzing the binding of a phosphorylated 2'-fluoropentose to a substrate base with the above purine nucleoside phosphorylase, so as to obtain the 2'-fluoronucleoside.

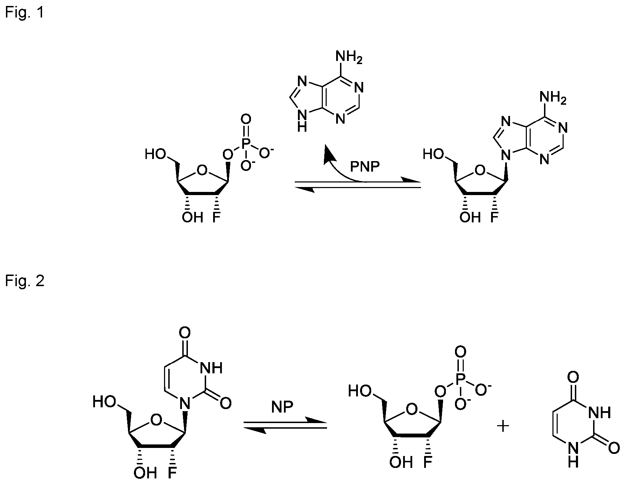

With the above method and the above purine nucleoside phosphorylase (PNP), the substrate base is used to substitute the phosphate group on the phosphorylated 2'-fluoropentose, thereby obtaining the 2'-fluoronucleoside combined with the substrate base. The above reaction (Step 2) is shown in Fig. 1, and the structures of the substrate base and phosphorylated 2'-fluoropentose include but are not limited to those shown in Fig. 1.

In a preferred embodiment, a method for preparing the phosphorylated 2'-fluoronucleoside includes: catalyzing the cleavage of a substrate nucleoside having a 2'-fluoronucleoside structure into a phosphorylated 2'-fluoropentose and a free base with a nucleoside phosphorylase; and preferably, the nucleoside phosphorylase includes but is not limited to a pyrimidine nucleoside phosphorylase, a thymidine phosphorylase, a uridine phosphorylase or a 5'-methylthioadenosine phosphorylase.

In the above method, the nucleoside phosphorylase includes but is not limited to a pyrimidine nucleoside phosphorylase derived from *Thermus thermophilus* as shown in SEQ ID NO: 1 or a mutant thereof (with 80% or more identity), and also includes nucleoside phosphorylases (NP) from other sources. The above reaction (Step 1) is shown in Fig. 2, and the structure of the substrate nucleoside having a 2'-fluoropentose includes but is not limited to those shown in Fig. 2.

The above-mentioned nucleoside phosphorylase includes but is not limited to pyrimidine nucleoside phosphorylase (PyNP), thymidine phosphorylase (TP), uridine phosphorylase (UP) or 5'-methylthioadenosine phosphorylase (MTAP, EC2.4.2.28), and also includes but is not limited to other enzymes with the same function in the prior art. In the process of preparing phosphorylated 2'-fluoropentose, the phosphorylase is selected according to the type of substrate nucleoside having a 2'-fluoropentose.

Different phosphorylases act on substrates with different structures. In this method, the type of nucleoside phosphorylase used can be flexibly selected according to the known affinity between nucleoside phosphorylases and substrates in the prior art (Kamel S, Thiele I, Neubauer P, et al. Thermophilic nucleoside phosphorylases: Their properties, characteristics and applications [J]. Biochimica et Biophysica Acta (BBA)-Proteins & Proteomics, 2019, 1868(2): 140304.). The above-mentioned reaction that catalyzes the substrate nucleoside having a 2'-fluoropentose into a phosphorylated 2'-fluoropentose can be carried out simultaneously with the reaction that catalyzes the phosphorylated 2'-fluoropentose into a 2'-fluoronucleoside, in the same container and under the same reaction conditions. Therefore, the above method can realize the preparation of the 2'-fluoronucleoside by the one-pot method. The above method has a higher conversion rate and greater efficiency, and can significantly reduce the amount of enzyme used, thereby lowering production costs, and is suitable for industrial scale-up production.

In a preferred embodiment, the substrate nucleoside includes but is not limited to a 2'-fluoro-2'-deoxyuridine, a 2'-fluoro-2'-deoxycytidine, a 2'-deoxy-2'-fluorothymidine, or other modified 2'-fluoronucleosides; and preferably, the substrate nucleoside includes a 2'-fluoro-2'-deoxyuridine.

In a preferred embodiment, the substrate base includes a modified or unmodified adenine, guanine, thymine, cytosine, or a modified uracil; and preferably, the substrate base includes an adenine, a guanine or a 2-aminoadenine.

In a preferred embodiment, the 2'-fluoronucleoside includes a nucleoside containing a 2'-fluoro-2'-deoxypentose structure, and preferably, the 2'-fluoronucleoside includes but is not limited to a 2'-fluoro-2'-deoxyadenosine, a 2'-fluoro-2'-deoxy-2-aminoadenosine, a 2'-fluoro-2'-deoxyguanosine or a 2'-fluoro-2'-deoxycytidine; or the 2'-fluoronucleoside is selected from a 2'-fluoro-2'-deoxynucleoside with a substituent on the base structure.

The substituent on the above base structure or the modification of the above substrate base includes but is not limited to introduction of methyl (-CH₃), carbamoyl (-CH₂NH₂), glycosyl, a phosphate group (-PO₄), thiol (-SH), nitro (-NO₂), hydroxyl (-OH) or deamination (-NH₂) on the base structure, thereby achieving modifications such as methylation, aminomethylation, glycosylation, phosphorylation, sulfuration, nitration, oxidation or deamination of the nucleoside.

Methylation: a modification of a base of a nucleoside by the addition of a methyl group. For example, in DNA, a cytosine base can undergo methylation to form 5-methylcytosine.

Aminomethylation: a modification of a base of a nucleoside by the addition of a carbamoyl group. This modification can occur on cytosine and adenine bases.

Glycosylation: a modification of a base of a nucleoside by the addition of a sugar moiety. For example, an adenine in a adenosine nucleotide can undergo glycosylation.

Phosphorylation: a modification of a base of a nucleoside by the addition of a phosphate group. Phosphorylation modification can occur on cytosine, adenine and guanine bases.

Sulfuration: a modification of a base of a nucleoside by the addition of a sulfur group. Sulfuration modification can occur on cytosine, adenine and guanine bases.

Nitration: a modification of a base of a nucleoside by the addition of a nitrate group. This modification can occur on cytosine and adenine bases.

Oxidation: a modification of a base of a nucleoside by the addition of an oxygen group. For example, in DNA, a guanine base undergoes oxidative modification to form 8-oxoguanine (8-oxoG).

Deamination: a modification of a base of a nucleoside by the loss of an amino group. This modification can occur on cytosine, adenine and guanine bases.

The beneficial effects of this application will be further explained in detail below in conjunction with specific examples.

### Example 1 Construction of TtPyNP expression strain

The protein sequence of pyrimidine nucleoside phosphorylase derived from *Thermus thermophilus* was obtained from NCBI (Accession No. BAD71594), as shown in SEQ ID NO: 1. After codon optimization, the DNA sequence encoding the enzyme was obtained, as shown in SEQ ID NO: 2, and cloned into the expression vector pET28a(+), resulting in the plasmid pET28a-TtPyNP. The pET28a-TtPyNP plasmid was transformed into *Escherichia coli* BL21(DE3) competent cells to obtain a monoclonal strain.
SEQ ID NO: 1: 
MNPVAFIREKREGKKHRREDLEAFLLGYLHDEVPDYQVSAWLMAAFLRGLDPEETLWLTETM
ARSGKVLDLSGLPHPVDQHSSGGVGDKVSLVVGPILAASGCTFAKMSGRTLAHTGGNIDKLESVP
 
GWRGEMTEAEFLERARRVGLVIAAQSPDLTPLDGKLYALRDVTATTESVPLIASSIMSKKLAVGARSI
VLDVKVGRGAFMKTLEEARLLAKTMVAIGQGDGRRVRALLTSMEAPLGRAVGNAIEVREAIEALKG
EGPGDLLEVALALAEEALRREGLDPALARKALEGGAALEKFRAFLEAQGGDPRAVEDFSLLPLAEE
HPLRAEREGVVREVDAYKVGLAVLALGGGRKRKGEPIDHGVGVYLLKKPGDRVERGEALALVYHR
RRGLEEALGHLREAYALGEEAHPAPLVLEAI.
SEQ ID NO: 2:

ATGAACCCGGTGGCGTTTATTCGCGAAAAACGCGAAGGCAAGAAACATCGCCGCGAGGA
TCTGGAAGCGTTTCTGCTGGGCTATCTGCACGATGAAGTGCCGGATTATCAGGTGAGCGCGTG
GCTGATGGCGGCGTTTTTACGCGGCTTAGATCCGGAAGAAACCCTGTGGCTGACCGAAACGA
TGGCCCGCAGCGGTAAAGTGCTGGATCTGAGCGGTCTGCCGCATCCGGTTGATCAGCATAGC
AGCGGCGGCGTGGGCGATAAAGTGAGCTTAGTGGTGGGCCCGATTCTGGCGGCGAGCGGTT
GTACCTTTGCGAAAATGTCGGGCCGCACCCTGGCGCATACCGGTGGTAACATTGATAAACTGG
AAAGCGTGCCGGGCTGGCGCGGCGAAATGACCGAAGCAGAATTTCTGGAACGCGCCCGTCG
CGTTGGTCTGGTTATTGCGGCGCAAAGCCCGGATTTAACGCCGTTAGATGGCAAACTGTATGC
GCTGCGCGATGTGACCGCGACCACTGAATCAGTTCCGCTGATTGCGAGCAGCATTATGAGCAA
GAAACTGGCGGTGGGCGCCCGTAGCATTGTGTTAGATGTTAAAGTGGGCCGCGGCGCGTTTA
TGAAAACCCTGGAAGAAGCGCGTCTGCTGGCGAAAACGATGGTGGCGATTGGCCAGGGCGA
TGGCCGTCGTGTTCGTGCGTTATTAACCAGCATGGAAGCGCCTCTGGGTCGTGCGGTTGGCA
ATGCGATTGAAGTGCGTGAAGCGATTGAAGCGCTGAAAGGCGAAGGCCCGGGCGATTTATTA
GAAGTGGCGCTGGCGTTAGCGGAAGAAGCGCTGCGTCGTGAAGGCTTAGATCCGGCGCTGG
CGCGTAAAGCGTTAGAAGGTGGCGCGGCGCTGGAAAAATTTCGCGCGTTTCTGGAAGCGCAG
GGCGGCGATCCGCGTGCAGTTGAAGATTTTAGCCTGCTGCCGCTGGCGGAAGAACATCCGTT
ACGTGCGGAACGCGAAGGCGTGGTTCGCGAAGTTGATGCGTATAAAGTGGGCCTGGCGGTG
CTGGCGTTAGGCGGTGGTCGTAAACGTAAAGGTGAACCGATTGATCATGGCGTGGGCGTGTAT
CTGCTGAAGAAACCGGGCGATCGCGTGGAACGCGGCGAAGCGTTAGCGTTAGTGTATCATCG
CCGCCGCGGCTTAGAAGAAGCGCTGGGTCATTTACGCGAAGCGTATGCGCTGGGCGAAGAAG
CGCATCCGGCGCCTCTGGTTTTAGAAGCGATTTAA.

### Example 2 Construction of GsPNP expression strain

The protein sequence of purine nucleoside phosphorylase derived from *Geobacillus stearothermophilus* was obtained from NCBI (Accession No. BAA13510), as shown in SEQ ID NO: 3. After codon optimization, the DNA sequence encoding the enzyme was obtained, as shown in SEQ ID NO: 4, and cloned into the expression vector pET28a(+), resulting in the plasmid pET28a-GsPNP. The pET28a-GsPNP plasmid was transformed into *Escherichia coli* BL21(DE3) competent cells to obtain a monoclonal strain.
SEQ ID NO: 3: 
MSVHIGAKEHEIADKILLPGDPLRAKYIAETFLEGATCYNQVRGMLGFTGTYKGHRISVQGTG
MGVPSISIYITELMQSYNVQTLIRVGTCGAIQKDVKVRDVILAMTSSTDSQMNRMTFGGIDYAPTAN
FDLLKTAYEIGKEKGLQLKVGSVFTADMFYNENAQFEKLARYGVLAVEMETTALYTLAAKFGRKALS
VLTVSDHILTGEETTAEERQTTFNEMIEVALETAIRQ.
SEQ ID NO: 4:

ATGAGCGTGCATATTGGCGCGAAAGAACATGAAATTGCGGATAAAATTCTGCTGCCGGGC
GATCCGTTACGCGCGAAATATATTGCGGAAACCTTTCTGGAAGGCGCGACCTGCTATAACCAG
GTGCGCGGCATGCTGGGCTTTACCGGCACCTATAAAGGCCATCGCATTAGCGTGCAGGGCAC
CGGCATGGGCGTTCCTAGCATTTCAATTTATATTACCGAACTGATGCAGAGCTATAACGTGCAG
ACCCTGATTCGCGTGGGCACCTGCGGTGCGATTCAAAAAGATGTGAAAGTGCGCGATGTGATT
CTGGCGATGACCAGCAGCACCGATAGCCAGATGAACCGCATGACCTTTGGCGGCATTGATTAT
GCGCCGACCGCGAACTTTGATCTGCTGAAAACCGCGTATGAAATTGGCAAAGAAAAAGGCCT
GCAGCTGAAAGTGGGCAGCGTGTTTACCGCGGATATGTTTTATAACGAAAACGCGCAGTTTGA
GAAACTGGCCCGCTATGGCGTGCTGGCGGTTGAAATGGAAACCACCGCGCTGTATACCCTGG
CGGCGAAATTTGGCCGCAAAGCGCTGAGCGTGCTGACCGTGAGCGATCATATTCTGACCGGC
GAAGAAACCACCGCGGAAGAACGCCAGACCACCTTTAACGAAATGATTGAAGTGGCGCTGGA
AACCGCGATTCGCCAGTAA.

### Example 3 Preparation of enzyme solution

Protein expression: wild-type and mutant seed cultures were inoculated into test tubes containing 5 mL Luria-Bertani liquid medium (containing 50 µg/mL kanamycin), cultured at 37°C for 16 h, and then inoculated at a 1% inoculum volume into a 2 L shake flask containing 500 mL of Luria-Bertani liquid medium. When culturing at 37°C until OD₆₀₀ reached 0.6, IPTG was added at a final concentration of 0.1 mM to induce expression, followed by induction at 20°C for 18 h. The cultured bacterial solution was centrifuged at 7000 rpm for 10 min to collect the bacterial cells, which were stored at -20°C for later use.

Preparation of enzyme solution: 0.1 g of the bacterial sludge prepared above was weighed and resuspended in 1 mL of 2 mM potassium phosphate buffer (pH 7.5). After shaking and mixing, the bacterial cell suspension was disrupted with an ultrasonic disruptor (power: 30%, time: 5 min).

HPLC detection method: after the reaction was completed, an equal volume of DMSO was added to terminate the reaction. The mixture was diluted twice before HPLC detection was performed. Chromatographic column: Atlantis T3 Column, 4.6 mm × 150 mm; mobile phase: 0.1% TFA/methanol; flow rate: 1 mL/min; 40°C; UV detector; detection wavelength: 254 nm; detection duration: 15 min.

### Example 4 Use of GsPNP mutants in synthesis of 2'-fluoro-2'-deoxyadenosine

Site-directed mutagenesis and multi-site mutagenesis were performed using GsPNP as shown in SEQ ID NO: 3 as the template. Reaction conditions: 100 mM 2'-fluoro-2'-deoxyuridine, 150 mM adenine, 807 µL TtPyNP crude enzyme solution, 1291 µL GsPNP mutant crude enzyme solution, 2 mM PBS (pH=7.0), 3 mL, 60°C, 200 rpm, 20 h. The conversion rate of 2'-fluoro-2'-deoxyuridine is shown in Table 1.

**Table 1**

| Mutant | Step 1 conve rsion rate | Step 2 conver sion rate |
|---|---|---|
| GsPNP | - | # |
| P19M, P19K, Q41D, Q41G, Q41N, Q41T, T51F, T51W, T51Y, R56K, T62C, I69M, S70G, 173A, 173G, I73L, T74Q, Q78A, Q78K, S79T, Y80A, Y80P, Y80T, N81G, V82A, Q83G, A93S, I94A, I94L, I94N, K96E, K96P, K96S, V98E, V98H, V98L, V98M, V98Q, V98S, V98T, K99S, M107E, M107G, M107H, M107N, M107S, M107Y, T108I, T108L, T108R, T108V, S109N, S113N, T119C, T119K, Y125F, Y125H, Y125K, Y125M, Y125Y, A126G, A126S, T128C, N130D, F131A, F131I, F131L, F131P, F131S, F131T, F131W, K135E, Y138S, Y138L, K142R, K144A, K144L, L148A, L148G, L148P, L148Q, L148S, L148Y, V150E, V150P, S152D, S152G, S152H, S152K, S152N, S152P, V153S, F154L, F154K, A156E, F166I, F166P, F166S, F166T, F166V, K168E, A170E, A170R, Y172A, Y172C, Y172G, Y172L, Y172N, Y172Q, Y172R, V174D, V174G, V174L, V174M, V174N, V174Q, V174S, V174T, L175A, L175E, L175G, L175K, L175N, L175S, L175T, 1205A, 1205V, L2061, L148P+P19M, L148P+P19K, L148P+Q41D, L148P+Q41G, L148P+Q41N, L148P+Q41T, L148P+T51F, L148P+T51W, L148P+T51Y, L148P+R56K, L148P+T62C, L148P+I69M, L148P+S70G, L148P+I73A, L148P+I73G, L148P+I73L, L148P+T74Q, L148P+Q78A, L148P+Q78K, L148P+S79T, L148P+Y80A, L148P+Y80P, L148P+Y80T, L148P+N81G, L148P+V82A, L148P+Q83G, L148P+A93S, L148P+I94A, L148P+I94L, L148P+194N, L148P+K96E, L148P+K96P, L148P+K96S, L148P+V98E, | + | * |
| L148P+V98H, L148P+V98L, L148P+V98M, L148P+V98Q, L148P+V98S, L148P+V98T, L148P+K99S, L148P+M107E, L148P+M107G, L148P+M107H, L148P+M107N, L148P+M107S, L148P+M107Y, L148P+T108I, L148P+T108L, L148P+T108R, L148P+T108V, L148P+S109N, L148P+S113N, L148P+T119C, L148P+T119K, L148P+Y125F, L148P+Y125H, L148P+Y125K, L148P+Y125M, L148P+Y125Y, L148P+A126G, L148P+A126S, L148P+T128C, L148P+N130D, L148P+F131A, L148P+F1311, L148P+F131L, L148P+F131P, L148P+F131S, L148P+F131T, L148P+F131W, L148P+K135E, L148P+Y138S, L148P+Y138L, L148P+K142R, L148P+K144A, L148P+K144L, L148P+V150E, L148P+V150P, L148P+S152D, L148P+S152G, L148P+S152H, L148P+S152K, L148P+S152N, L148P+S152P, L148P+V153S, L148P+F154L, L148P+F154K, L148P+A156E, L148P+F166I, L148P+F166P, L148P+F166S, L148P+F166T, L148P+F166V, L148P+K168E, L148P+A170E, L148P+A170R, L148P+Y172A, L148P+Y172C, L148P+Y172G, L148P+Y172L, L148P+Y172N, L148P+Y172Q, L148P+Y172R, L148P+V174D, L148P+V174G, L148P+V174L, L148P+V174M, L148P+V174N, L148P+V174Q, L148P+V174S, L148P+V174T, L148P+L175A, L148P+L175E, L148P+L175G, L148P+L175K, L148P+L175N, L148P+L175S, L148P+L175T, L148P+I205A, L148P+1205V, L148P+L206I | | |
| L148P+P19K+M107G, L148P+P19M+F166P, L148P+Q41G+T119K, L148P+Q41D+A156E, L148P+Q41N+K96E, L148P+Q41T+T51W, L148P+Q41T+N81G, L148P+Q41T+T108R, L148P+Q41T+L175K, L148P+T51F+F166I, L148P+T51W+V174S, L148P+T51Y+F166P, L148P+R56K+A126S, L148P+T62C+M107H, L148P+I69M+K135E, L148P+S70G+Y125H, L148P+173A+S152N, L148P+I73A+M107Y, L148P+I73G+V98Q, L148P+T74Q+I94N, L148P+Q78A+V174S, L148P+Q78K+L175G, L148P+S79T+A93S, L148P+Y80A+F154L, L148P+Y80P+Y172A, L148P+Y80T+L206I, L148P+Q83G+S152D, L148P+V98E+V153S, L148P+V98L+V174L, L148P+V98M+F131I, L148P+V98H+K168E, L148P+V98S+Y172Q, L148P+M107S+S152K, L148P+T108L+V174D, L148P+T108V+L175K, L148P+S113N+S152H, L148P+T119C+S152G, L148P+Y125M+Y172L, L148P+A126G+Y172G, L148P+T128C+V174N, L148P+N130D+V150E, L148P+F131L+I205V, L148P+F131P+Y172C, L148P+F131W+L175S, L148P+Y138S+V174Q, L148P+F166S+V174M, L148P+F166V+L175E | ++ | ** |
| L148P+P19K+T108V+Y125Y, L148P+P19M+M107N+F166P, L148P+Q41D+M107G+S152P, L148P+Q41N+Y80P+K144A, L148P+Q41T+M107H+V174L, L148P+Q41T+L175K+I73A, L148P+T51W+Y80P+V174S, L148P+T51Y+F131T+V174G, L148P+R56K+Q78A+F166S, L148P+T62C+Y125K+V150P, | +++ | *** |
| L148P+173A+T108L+F131S, L148P+I73G+K96P+S152H, L148P+I73L+V98M+F166P, L148P+V82A+A93S+F131L, L14SP+I69M+A156E+L175E, L148P+S70G+S113N+Y172R, L148P+S79T+A126G+S152D, L148P+194A+F131P+V174M, L148P+I94L+Y138L+V174T, L148P+I94N+N130D+L206I, L148P+K96E+F131W+F166I, L148P+V98H+T119K+F166T, L148P+V98Q+Y138S+A170E, L148P+V98L+K135E+V174L, L148P+M107E+S152N+L175K, L148P+T108R+A126S+K168E, L148P+S109N+F1311+F154L, L148P+T119C+Y125H+K135E, L148P+F131A+K142R+V174S, L148P+S152G+F166V+1205V | | |
| L148P+P19K+K96P+V150P+A126S, L148P+P19M+M107N+F166P+V174T, L148P+Q41D+T108L+K144A+V153S, L148P+Q41G+T51Y+S113N+F166, L148P+Q41N+T51F+A93S+F131A, L148P+Q41N+Y80A+S152G+L175E, L148P+Q41T+Y80A+V150E+Y172A, L148P+Q41T+I73A+S113N+L175K, L148P+T51W+Y80P+Y125M+V174S, L148P+T51Y+173A+Y125K+L142R, L148P+R56K+M107H+A126G+L206I, L148P+T62C+V98Q+M107G+S152P, L148P+I69M+F131W+L175K+L206I, L148P+173G+F131L+S152K+Y172C, L148P+I73L+I94A+T119C+S152D, L148P+T74Q+V98S+T108I+I205V, L148P+Q78A+S152P+F166V+L175G, L148P+Q78K+194L+L175E+L206I, L148P+S79T+K96E+Y138L+V174M, L148P+Y80T+M107S+S152H+Y172R, L148P+N81G+Y125Y+F131T+A156E, L148P+Q83G+M107Y+A126S+K168E, L148P+V98L+T119K+K135E+V174L, L148P+V98T+M107E+Y138S+I205A, L148P+Y125H+F131I+S152H+L175T | ++++ | **** |
| L148P+P19M+T51Y+M107N+F166P+V174T, L148P+Q41T+173A+A126S+L175K+S113N, L148P+T51W+Y80P+Y125M+S152P+V174S, L148P+I73L+Y80T+M107S+S152H+Y172R, L148P+Q78A+S152P+F166V+L175G+I205V, L148P+V98L+T119K+K135E+F166P+V174L, L148P+P19M+T51Y+M107N+S152H+F166P+V174T, L148P+Q41D+V9SL+T119K+K135E+F166P+V174L, L148P+Q41T+I73A+A126S+K144A+L175K+S113N, L148P+T51W+Y80P+M107N+S113N+F166V+I205A, L148P+T51W+Y80P+Y125M+S152P+V174S+L206I, L148P+R56K+I73L+Y80T+M107S+S152H+Y172R, L148P+Q78A+M107S+S152P+F166V+L175G+I205V, L148P+P19M+T51Y+I73A+M107N+S152H+F166P+V174T, L148P+Q41D+V98L+T119K+K135E+S152H+F166P+V174L, L148P+Q41T+173A+S113N+A126S+K144A+L175K+I205V, | ++++ + | ***** |
| L148P+T51W+Y80P+M107N+S113N+F166V+L175G+I205A, L148P+T51W+Y80P+V98L+Y125M+S152P+V174S+L206I, L148P+R56K+I73L+Y80T+M107S+T119K+S152H+Y172R, L148P+Q78A+M107S+Y125H+S152P+F166V+L175G+I205V, L148P+P19M+Q41T+I73A+S113N+A126S+K144A+L175K+I205V, L148P+P19M+T51Y+173A+V98T+M107N+S152H+F166P+V174T, L148P+Q41D+V98L+T119K+K135E+S152H+F166P+V174L+I205V, L148P+T51W+Y80P+V98T+M107N+S113N+F166V+L175G+I205A, L148P+T51W+Y80P+V98L+Y125M+S152P+F166V+V174S+L206I, L148P+R56K+I73L+Y80T+M107S+T119K+S152H+Y172R+L206I, L148P+Q78A+M107S+S113N+Y125H+S152P+F166V+L175G+I205V, L148P+P19M+Q41T+I69M+I73A+S113N+A126S+K144L+L175K+I205V, L148P+P19M+T51Y+I73A+V98T+M107N+S152H+F166P+V174T+L206I, L148P+Q41D+S70G+V98L+T119K+K135E+S152H+F166P+V174L+I205V, L148P+T51W+I69M+Y80P+V98T+M107N+S113N+F166V+L175G+I205A, L148P+T51W+Y80P+V98L+Y125M+K144L+S152P+F166V+V174S+L206I, L148P+R56K+I73L+Y80T+M107S+T119K+K144A+S152H+Y172R+L206I, L148P+Q78A+V98L+M107S+S113N+Y125H+S152P+F166V+L175G+I205V | | |
| L148P+P19M+Q41T+169M+173A+S113N+Y125F+A126S+T128C+N130D+K144 L+L175K+I205V, L148P+P19M+Q41D+T51Y+I73A+V98T+M107N+A126G+K135E+S152H+F166 P+V174T+L206I, L148P+Q41D+S70G+V98L+T108L+T119K+Y125F+K135E+K144A+S152H+F1 66P+V174L+1205V, L148P+T51W+I69M+Y80P+V98T+M107N+S113N+N130D+K142R+F154L+F16 6V+L175G+I205A, L148P+T51W+T74Q+Y80P+V98L+T108I+S113N+Y125M+K144L+S152P+F166 V+V174S+L206I, L148P+R56K+173L+Y80T+V98M+M107S+T119K+Y125F+F131S+K144A+S152 H+Y172R+L206I, L148P+I69M+Q78A+V98L+M107S+S113N+Y125H+N130D+K144A+S152P+F1 66V+L175G+I205V | ++++ ++ | ****** |

| | | |
|---|---|---|
| Note: in the above table, the "-" represents a conversion rate of 0.1-10% (excluding the endpoint value of 10%), the "+" represents a conversion rate of 10-20% (excluding the endpoint value of 20%), the "++" represents a conversion rate of 20-30% (excluding the endpoint value of 30%), the "+++" represents a conversion rate of 30-40% (excluding the endpoint value of 40%), the "++++" represents a conversion rate of 40-50% (excluding the endpoint value of 50%), the "+++++" represents a conversion rate of 50-70%, and the "++++++" represents a conversion rate of >70%. | | |

The "#" represents a conversion rate of 0.1-5% (excluding the endpoint value of 5%), the "*" represents a conversion rate of 5-10% (excluding the endpoint value of 10%), the "**" represents a conversion rate of 10-20% (excluding the endpoint value of 20%), the "***" represents a conversion rate of 20-30% (excluding the endpoint value of 30%), the "****" represents a conversion rate of 30-40% (excluding the endpoint value of 40%), the "*****" represents a conversion rate of 40-50%, and the "******" represents a conversion rate of >50%.

In the above Step 1 + Step 2 reactions, the calculation of conversion rate is based on the conversion rate of the substrate with the lower concentration. Since 2'-fluoro-2'-deoxyuridine is present in smaller amounts, enzyme activity is evaluated by assessing the conversion rate of Step 1.

In the above Step 1 + Step 2 reactions, the 2 reactions are reversible. Therefore, as the enzyme activity of the GsPNP mutant increases, the conversion rate of Step 2 increases, and the concentration of the reaction intermediate, phosphorylated 2'-fluoropentose, decreases. This promotes the forward progression of Step 1, resulting in a corresponding increase in the conversion rate of Step 1.

### Example 5 Reaction scale-up

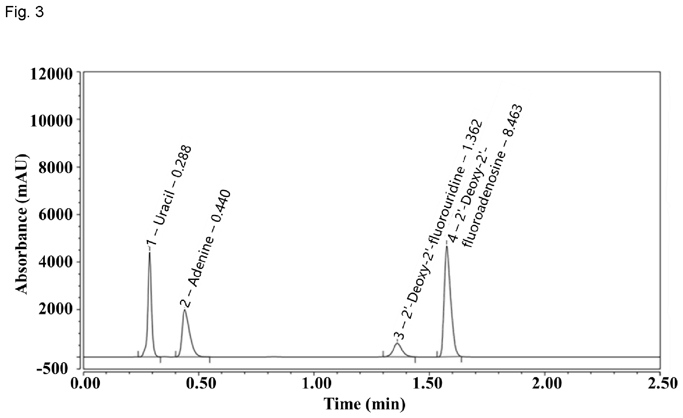

In a 20 mM phosphate buffer (pH=7.5), a 2 mL reaction system was prepared, which included 200 mM 2'-fluoro-2'-deoxyuridine, 250 mM adenine, 430 µL of 3-fold concentrated TtPyNP crude enzyme solution, and 258 µL of 3-fold concentrated L148P+I69M+Q78A+V98L+M107S+S113N+Y125H+N130D+K144A+S152P+F166V+L175G+I205 V crude enzyme solution. The reaction was carried out at 60°C for 159 h. After the reaction was completed, an equal volume of DMSO was added for dissolution. After being diluted 50 times, the mixture was sent for HPLC detection. The results are as shown in Fig. 3. The Step 1 conversion rate of 2'-deoxy-2'-fluorouridine was greater than 81%, the Step 2 conversion rate of adenine was greater than 63%, and the yield of 2'-fluoro-2'-deoxyadenosine (2'-deoxy-2'-fluoroadenosine) was 42.63 g/L.

From the above description, it can be seen that the above examples of the present application achieve the following technical effects: the binding of a phosphorylated 2'-fluoropentose and a substrate base can be efficiently catalyzed with the above purine nucleoside phosphorylase mutant to prepare and obtain the 2'-fluoronucleoside. Furthermore, the cleavage of a substrate nucleoside having a 2'-fluoropentose into a phosphorylated 2'-fluoropentose and a free base can be catalyzed with nucleoside phosphorylases such as pyrimidine nucleoside phosphorylase, thymidine phosphorylase, uridine phosphorylase or 5'-methylthioadenosine phosphorylase in the prior art, thereby providing the reaction substrate for the catalytic reaction of the above purine nucleoside phosphorylase mutant. Therefore, the above two-step catalytic reactions are carried out at the same time, in the same container and under the same reaction conditions to prepare and obtain the 2'-fluoronucleoside with the one-pot method. Compared with the wild-type enzyme, the above purine nucleoside phosphorylase mutant has a better enzyme activity, and a higher conversion rate and greater efficiency in preparing 2'-fluoronucleoside products. It can significantly reduce the amount of enzyme used, thereby lowering production costs, and is suitable for industrial scale-up production.

The above descriptions are merely preferred examples of the present application and are not intended to limit the present application. For person skilled in the art, various modifications and changes may be made to the present application. Any modification, equivalent replacement, improvement, etc. made within the spirit and principles of the present application shall be included within the scope of protection of the present application.

## Claims

1. A purine nucleoside phosphorylase mutant, comprising:
(a) a protein with a mutation based on the purine nucleoside phosphorylase wild-type enzyme GsPNP as shown in SEQ ID NO: 3, wherein the mutation is selected from any one or more of the following mutations: L148, P19, Q41, T51, R56, T62, I69, S70, I73, T74, Q78, S79, Y80, N81, V82, Q83, A93, I94, K96, V98, K99, M107, T108, S109, S113, T119, Y125, A126, T128, N130, F131, K135, Y138, L142, K144, V150, S152, V153, F154, A156, F166, K168, A170, Y172, V174, L175, I205 or L206; or
(b) a protein that has greater than 70% identity to the amino acid sequence as defined in (a) and has a purine nucleoside phosphorylase activity.

2. The purine nucleoside phosphorylase mutant according to claim 1, wherein the mutation is selected from any one or more of the following mutations:
L148P, P19M, P19K, Q41D, Q41G, Q41N, Q41T, T51F, T51W, T51Y, R56K, T62C, I69M, S70G, 173A, 173G, I73L, T74Q, Q78A, Q78K, S79T, Y80A, Y80P, Y80T, N81G, V82A, Q83G, A93S, I94A, 194L, 194N, K96E, K96P, K96S, V98E, V98H, V98L, V98M, V98Q, V98S, V98T, K99S, M107E, M107G, M107H, M107N, M107S, M107Y, T108I, T108L, T108R, T108V, S109N, S113N, T119C, T119K, Y125F, Y125H, Y125K, Y125M, Y125Y, A126G, A126S, T128C, N130D, F131A, F1311, F131L, F131P, F131S, F131T, F131W, K135E, Y138S, Y138L, K142R, K144A, K144L, L148A, L148G, L148Q, L148S, L148Y, V150E, V150P, S152D, S152G, S152H, S152K, S152N, S152P, V153S, F154L, F154K, A156E, F166I, F166P, F166S, F166T, F166V, K168E, A170E, A170R, Y172A, Y172C, Y172G, Y172L, Y172N, Y172Q, Y172R, V174D, V174G, V174L, V174M, V174N, V174Q, V174S, V174T, L175A, L175E, L175G, L175K, L175N, L175S, L175T, I205A, 1205V or L2061,
wherein a letter in front of a number represents an original amino acid, and a letter behind the number represents a mutated amino acid.

3. The purine nucleoside phosphorylase mutant according to claim 1, wherein the mutation comprises any one of the following amino acid mutations:
L148P+P19M, L148P+P19K, L148P+Q41D, L148P+Q41G, L148P+Q41N, L148P+Q41T, L148P+T51F, L148P+T51W, L148P+T51Y, L148P+R56K, L148P+T62C, L148P+I69M, L148P+S70G, L148P+173A, L148P+I73G, L148P+173L, L148P+T74Q, L148P+Q78A, L148P+Q78K, L148P+S79T, L148P+Y80A, L148P+Y80P, L148P+Y80T, L148P+N81G, L148P+V82A, L148P+Q83G, L148P+A93S, L148P+I94A, L148P+I94L, L148P+I94N, L148P+K96E, L148P+K96P, L148P+K96S, L148P+V98E, L148P+V98H, L148P+V98L, L148P+V98M, L148P+V98Q, L148P+V98S, L148P+V98T, L148P+K99S, L148P+M107E, L148P+M107G, L148P+M107H, L148P+M107N, L148P+M107S, L148P+M107Y, L148P+T108I, L148P+T108L, L148P+T108R, L148P+T108V, L148P+S109N, L148P+S113N, L148P+T119C, L148P+T119K, L148P+Y125F, L148P+Y125H, L148P+Y125K, L148P+Y125M, L148P+Y125Y, L148P+A126G, L148P+A126S, L148P+T128C, L148P+N130D, L148P+F131A, L148P+F131I, L148P+F131L, L148P+F131P, L148P+F131S, L148P+F131T, L148P+F131W, L148P+K135E, L148P+Y138S, L148P+Y138L, L148P+K142R, L148P+K144A, L148P+K144L, L148P+V150E, L148P+V150P, L148P+S152D, L148P+S152G, L148P+S152H, L148P+S152K, L148P+S152N, L148P+S152P, L148P+V153S, L148P+F154L, L148P+F154K, L148P+A156E, L148P+F166I, L148P+F166P, L148P+F166S, L148P+F166T, L148P+F166V, L148P+K168E, L148P+A170E, L148P+A170R, L148P+Y172A, L148P+Y172C, L148P+Y172G, L148P+Y172L, L148P+Y172N, L148P+Y172Q, L148P+Y172R, L148P+V174D, L148P+V174G, L148P+V174L, L148P+V174M, L148P+V174N, L148P+V174Q, L148P+V174S, L148P+V174T, L148P+L175A, L148P+L175E, L148P+L175G, L148P+L175K, L148P+L175N, L148P+L175S, L148P+L175T, L148P+I205A, L148P+1205V, L148P+L206I, L148P+P19K+M107G, L148P+P19M+F166P, L148P+Q41G+T119K, L148P+Q41D+A156E, L148P+Q41N+K96E, L148P+Q41T+T51W, L148P+Q41T+N81G, L148P+Q41T+T108R, L148P+Q41T+L175K, L148P+T51F+F166I, L148P+T51W+V174S, L148P+T51Y+F166P, L148P+R56K+A126S, L148P+T62C+M107H, L148P+I69M+K135E, L148P+S70G+Y125H, L148P+173A+S152N, L148P+I73A+M107Y, L148P+I73G+V98Q, L148P+T74Q+I94N, L148P+Q78A+V174S, L148P+Q78K+L175G, L148P+S79T+A93S, L148P+Y80A+F154L, L148P+Y80P+Y172A, L148P+Y80T+L206I, L148P+Q83G+S152D, L148P+V98E+V153S, L148P+V98L+V174L, L148P+V98M+F131I, L148P+V98H+K168E, L148P+V98S+Y172Q, L148P+M107S+S152K, L148P+T108L+V174D, L148P+T108V+L175K, L148P+S113N+S152H, L148P+T119C+S152G, L148P+Y125M+Y172L, L148P+A126G+Y172G, L148P+T128C+V174N, L148P+N130D+V150E, L148P+F131L+I205V, L148P+F131P+Y172C, L148P+F131W+L175S, L148P+Y138S+V174Q, L148P+F166S+V174M, L148P+F166V+L175E, L148P+P19K+T108V+Y125Y, L148P+P19M+M107N+F166P, L148P+Q41D+M107G+S152P, L148P+Q41N+Y80P+K144A, L148P+Q41T+M107H+V174L, L148P+Q41T+L175K+I73A, L148P+T51W+Y80P+V174S, L148P+T51Y+F131T+V174G, L148P+R56K+Q78A+F166S, L148P+T62C+Y125K+V150P, L148P+173A+T108L+F131S, L148P+I73G+K96P+S152H, L148P+I73L+V98M+F166P, L148P+V82A+A93S+F131L, L148P+I69M+A156E+L175E, L148P+S70G+S113N+Y172R, L148P+S79T+A126G+S152D, L148P+I94A+F131P+V174M, L148P+I94L+Y138L+V174T, L148P+I94N+N130D+L206I, L148P+K96E+F131W+F166I, L148P+V98H+T119K+F166T, L148P+V98Q+Y138S+A170E, L148P+V98L+K135E+V174L, L148P+M107E+S152N+L175K, L148P+T108R+A126S+K168E, L148P+S109N+F131I+F154L, L148P+T119C+Y125H+K135E, L148P+F131A+K142R+V174S, L148P+S152G+F166V+I205V, L148P+P19K+K96P+V150P+A126S, L148P+P19M+M107N+F166P+V174T, L148P+Q41D+T108L+K144A+V153S, L148P+Q41G+T51Y+S113N+F166I, L148P+Q41N+T51F+A93S+F131A, L148P+Q41N+Y80A+S152G+L175E, L148P+Q41T+Y80A+V150E+Y172A, L148P+Q41T+I73A+S113N+L175K, L148P+T51W+Y80P+Y125M+V174S, L148P+T51Y+I73A+Y125K+L142R, L148P+R56K+M107H+A126G+L206I, L148P+T62C+V98Q+M 107G+S152P, L148P+I69M+F131W+L175K+L206I, L148P+173G+F131L+S152K+Y172C, L148P+I73L+194A+T119C+S152D, L148P+T74Q+V98S+T108I+I205V, L148P+Q78A+S152P+F166V+L175G, L148P+Q78K+194L+L175E+L206I, L148P+S79T+K96E+Y138L+V174M, L148P+Y80T+M107S+S152H+Y172R, L148P+N81G+Y125Y+F131T+A156E, L148P+Q83G+M107Y+A126S+K168E, L148P+V98L+T119K+K135E+V174L, L148P+V98T+M107E+Y138S+I205A, L148P+Y125H+F131I+S152H+L175T, L148P+P19M+T51Y+M107N+F166P+V174T, L148P+Q41T+I73A+A126S+L175K+S113N, L148P+T51W+Y80P+Y125M+S152P+V174S, L148P+I73L+Y80T+M107S+S152H+Y172R, L148P+Q78A+S152P+F166V+L175G+I205V, L148P+V98L+T119K+K135E+F166P+V174L, L148P+P19M+T51Y+M107N+S152H+F166P+V174T, L148P+Q41D+V9SL+T119K+K135E+F166P+V174L, L148P+Q41T+I73A+A126S+K144A+L175K+S113N, L148P+T51W+Y80P+M107N+S113N+F166V+I205A, L148P+T51W+Y80P+Y125M+S152P+V174S+L206I, L148P+R56K+I73L+Y80T+M107S+S152H+Y172R, L148P+Q78A+M107S+S152P+F166V+L175G+I205V, L148P+P19M+T51Y+I73A+M107N+S152H+F166P+V174T, L148P+Q41D+V98L+T119K+K135E+S152H+F166P+V174L, L148P+Q41T+173A+S113N+A126S+K144A+L175K+I205V, L148P+T51W+Y80P+M107N+S113N+F166V+L175G+I205A, L148P+T51W+Y80P+V98L+Y125M+S152P+V174S+L206I, L148P+R56K+173L+Y80T+M107S+T119K+S152H+Y172R, L148P+Q78A+M107S+Y125H+S152P+F166V+L175G+I205V, L148P+P19M+Q41T+I73A+S113N+A126S+K144A+L175K+I205V, L148P+P19M+T51Y+I73A+V98T+M107N+S152H+F166P+V174T, L148P+Q41D+V98L+T119K+K135E+S152H+F166P+V174L+I205V, L148P+T51W+Y80P+V98T+M107N+S113N+F166V+L175G+I205A, L148P+T51W+Y80P+V98L+Y125M+S152P+F166V+V174S+L206I, L148P+R56K+173L+Y80T+M107S+T119K+S152H+Y172R+L206I, L148P+Q78A+M107S+S113N+Y125H+S152P+F166V+L175G+I205V, L148P+P19M+Q41T+I69M+I73A+S113N+A126S+K144L+L175K+I205V, L148P+P19M+T51Y+173A+V98T+M107N+S152H+F166P+V174T+L206I, L148P+Q41D+S70G+V98L+T119K+K135E+S152H+F166P+V174L+I205V, L148P+T51W+I69M+Y80P+V98T+M107N+S113N+F166V+L175G+I205A, L148P+T51W+Y80P+V98L+Y125M+K144L+S152P+F166V+V174S+L206I, L148P+R56K+I73L+Y80T+M107S+T119K+K144A+S152H+Y172R+L206I, L148P+Q78A+V98L+M107S+S113N+Y125H+S152P+F166V+L175G+I205V, L148P+P19M+Q41T+I69M+I73A+S113N+Y125F+A126S+T128C+N130D+K144L+L175K+I205V, L148P+P19M+Q41D+T51Y+I73A+VI8T+M107N+A126G+K135E+S1562H+F166P+V174T+L206I, L148P+Q41D+S70G+V98L+T108L+T119K+Y125F+K135E+K144A+S152H+F166P+V174L+1205V, L148P+T51W+169M+Y80P+V98T+M107N+S113N+N130D+K142R+F154L+F166V+L175G+I205A, L148P+T51W+T74Q+Y80P+V98L+T108I+S113N+Y125M+K144L+S152P+F166V+V174S+L206I, L148P+R56K+I73L+Y80T+VI8M+M107S+T119K+Y125F+F131S+K144A+S152H+Y172R+L206I, or L148P+I69M+Q78A+V98L+M107S+S113N+Y125H+N130D+K144A+S152P+F166V+L175G+I205 V.

4. The purine nucleoside phosphorylase mutant according to claim 1, wherein the purine nucleoside phosphorylase mutant comprises a protein as shown in SEQ ID NO: 5, or a protein that has greater than 80% identity to the protein as shown in SEQ ID NO: 5 and has a purine nucleoside phosphorylase activity.

5. A DNA molecule, wherein the DNA molecule encodes the purine nucleoside phosphorylase mutant according to any one of claims 1 to 4.

6. A recombinant plasmid, wherein the DNA molecule according to claim 5 is comprised in the recombinant plasmid.

7. A host cell, wherein the host cell comprises the DNA molecule according to claim 5 or the recombinant plasmid according to claim 6.

8. A method for preparing a 2'-fluoronucleoside, wherein the method comprises: catalyzing the binding of a phosphorylated 2'-fluoropentose to a substrate base with the purine nucleoside phosphorylase according to any one of claims 1 to 4, so as to obtain the 2'-fluoronucleoside.

9. The method according to claim 8, wherein a method for preparing the phosphorylated 2'-fluoronucleoside comprises: catalyzing the cleavage of a substrate nucleoside having a 2'-fluoronucleoside structure into a phosphorylated 2'-fluoropentose and a free base with a nucleoside phosphorylase.

10. The method according to claim 9, wherein the nucleoside phosphorylase comprises a pyrimidine nucleoside phosphorylase, a thymidine phosphorylase, a uridine phosphorylase or a 5'-methylthioadenosine phosphorylase.

11. The method according to claim 9, wherein the substrate nucleoside comprises a 2'-fluoro-2'-deoxyuridine, a 2'-fluoro-2'-deoxycytidine or a 2'-deoxy-2'-fluorothymidine.

12. The method according to claim 8, wherein the substrate base comprises a modified or unmodified adenine, guanine, thymine or cytosine, or a modified uracil.

13. The method according to claim 12, wherein the substrate base comprises an adenine, a guanine, or a 2-aminoadenine.

14. The method according to any one of claims 8 to 13, wherein the 2'-fluoronucleoside comprises a nucleoside comprising a 2'-fluoro-2'-deoxypentose structure.

15. The method according to claim 14, wherein the 2'-fluoronucleoside comprises a 2'-fluoro-2'-deoxyadenosine, a 2'-fluoro-2'-deoxy-2-aminoadenosine, a 2'-fluoro-2'-deoxyguanosine or a 2'-fluoro-2'-deoxycytidine; or
the 2'-fluoronucleoside is selected from a 2'-fluoro-2'-deoxynucleoside with a substituent on the base structure.
